Europäisches Patentamt

European Patent Office

Office européen des brevets

(11) Publication number: **0 151 869**

**B1**

(12) **EUROPEAN PATENT SPECIFICATION**

(45) Date of publication of patent specification: **31.01.90**

(51) Int. Cl.⁵: **A 61 F 9/00**

(21) Application number: **84307972.4**

(22) Date of filing: **16.11.84**

(54) Apparatus for ophthalmological surgery.

(30) Priority: **17.11.83 US 552983**

(43) Date of publication of application:
**21.08.85 Bulletin 85/34**

(45) Publication of the grant of the patent:
**31.01.90 Bulletin 90/05**

(84) Designated Contracting States:
**BE CH DE FR GB IT LI NL**

(56) References cited:
**EP-A-0 083 494**
**EP-A-0 111 060**
**DE-A-3 148 748**
**DE-B-1 288 245**
**SU-A- 782 810**
**US-A-3 769 963**
**US-A-3 982 541**
**US-A-4 173 980**
**US-A-4 336 809**
**US-A-4 461 294**

**LASER FOCUS, vol. 18, no. 1, January 1982,
pages 57-58, H.T: POWELL:"Excimers"**

**The file contains technical information
submitted after the application was filed and
not included in this specification**

(73) Proprietor: **L'Esperance, Francis A.**
**255 Oakwood Road**
**Englewood New Jersey 07631 (US)**

(72) Inventor: **L'Esperance, Francis A.**
**255 Oakwood Road**
**Englewood New Jersey 07631 (US)**

(74) Representative: **Milhench, Howard Leslie et al
R.G.C. Jenkins & Co. 26 Caxton Street
London SW1H 0RJ (GB)**

(56) References cited:
**LASER UND ELEKTRO-OPTIK, vol. 10, no. 1,
March 1978, pages 14-16, A. PATAKI et al.: "The
Laser Micro Beam as a Tool for Tissue Sampling
in Biochemistry"**

**LASER FOCUS, vol. 20, no. 10, October 1984,
pages 104, 106, L: GOLDMAN: "Laser Surgery
and Medicine in the Next Decade"**

**REVUE DE PHYSIQUE APPLIQUEE, vol. 15, no. 9,
September 1980, pages 1417-1426, Paris, FR,
J.M. BRUNETAUD et al.: "Les applications
thérapeutiques des lasers"**

Courier Press, Leamington Spa, England.

## Description

The invention relates to that aspect of ophthalmological surgery which is concerned with operations upon the external surface of the cornea.

Operations of the character indicated include corneal transplants and keratotomies; such operations have traditionally required skilled manipulation of a cutting instrument. But, however keen the cutting edge, the mere entry of the edge into the surface of the cornea necessarily means a wedge-like lateral pressure against body cells displaced by the entry, on both sides of the entry. Such lateral pressure is damaging to several layers of cells on both sides of the entry, to the extent impairing the ability of the wound to heal, and resulting in the formation of scar tissue.

The $CO_2$ laser has been employed in an effort to minimize such surgical damage to cells on severed sides of a given cut, as in the case of operations to remove a local skin defect. The beam of such a laser is characterized by a particular infrared wavelength (10.6 microns), and controlled local ablation or incision of the cornea is achieved, without developing any lateral pressure upon cells adjacent to the margins of ablation. However, the operation is not performed without side effects, in that the ablation or incision is thermally achieved, through photocoagulation and/or photovaporization; cells adjacent the ablated or incised margin are charred. And even with lasers emitting in the visible spectrum, the effect is still largely thermal in nature. For example, for visible laser irradiation of the skin at about 532.0 nanometers (0.532 micron), namely, in the pea-green portion of the visible spectrum, histological examination reveals evidence of cellular dehydration (i.e., cellular retraction with formation of tissue clefts, pyknotic nuclei) at energy densities where ablation can be accomplished; thus, at an energy level needed for ablation or incision with such radiation, charring (cellular damage) is observed at the site of the incision and is an indication of substrate heating.

On the other hand, radiation at ultraviolet wavelengths is characterized by high photon energy, and this energy is greatly effective on impact with tissue, in that molecules of tissue are decomposed on photon impact, resulting in tissue ablation by photodecomposition. Molecules at the irradiated surface are broken into smaller volatile fragments without heating the remaining substrate; the mechanism of the ablation is photochemical, i.e., the direct breaking of intramolecular bonds. Photothermal and/or photocoagulation effects are neither characteristic nor observable in ablations at ultraviolet wavelengths, and cell damage adjacent the photodecomposed ablation is insignificant.

It is therefore the object of the present invention to provide an improved apparatus for performing ophthalmological surgery upon the anterior surface of the cornea.

According to the present invention there is provided sculpture apparatus for performing ophthalmological surgery on the optically used portion of the anterior surface of the cornea, said apparatus comprising:

laser means adapted for producing an output beam having a cross-section that at corneal impingement is small in relation to said optically used portion, said output beam being in the ultraviolet portion of the electromagnetic spectrum and being capable of achieving selective ablation of said anterior surface with penetration into the stroma to achieve a volumetric removal of corneal tissue by photodecomposition,

laser control means, including a microprocessor, for controlling the operation of said laser means so as to effect a predetermined operative procedure, said laser control means including means for scan deflection of said beam for selectively impinging the beam throughout a predetermined area within said optically used portion, the irradiated flux density and exposure time of laser-beam impingement at the cornea being such as to achieve for each scan an ablation depth which is but a fraction of the maximum depth of a required penetration; and

said laser control means being adapted, at least in part by the programming of said microprocessor, to effect successive scans within said predetermined area, whereby to achieve said required penetration as a result of said successive scans.

Described hereinafter is an exemplary corneal sculpture apparatus in accordance with the present invention which effectively fixes the position of an eye with respect to a scanning laser characterized by ultraviolet radiation, at an energy level capable of achieving controlled ablative photodecomposition of the cornea, namely, of the epithelium, Bowman's membrane, and stroma levels of the cornea. Irradiated flux density and exposure time are so controlled as to achieve desired depth of the ablation, which is a local sculpturing step, and the scanning action is coordinated to achieve desired ultimate surface change in the cornea. The scanning may be so controlled as to change the front surface of the cornea from a greater to a lesser spherical curvature, or from a lesser to a greater spherical curvature, thus effecting reduction in a myopic or in a hyperopic condition, without resort to a contact or other corrective auxiliary lens technique, in that the cornea becomes the corrective lens. The scanning may also be so controlled as to reduce astigmatism. Still further, the scanning may be so controlled as to excise corneal tissue uniformly over a precisely controlled area of the cornea for precision accommodation of a corneal transplant.

The response of a biological target to pulsed ultraviolet radiation emitted by a KrF excimer laser system was studied by J. Taboada et al and a corresponding report appears in "Health Physics", Vol. 40, May 1981, pp. 677 to 683 under the title "Response of the Corneal Epithelium to KrF Excimer Laser Pulses". A paper by the same author and entitled "Extreme Sensitivity in the Corneal Epithelium to Far UV ArF Excimer Laser Pulses" was presented to the Aerospace Medical Association in 1981 Meeting in San Antonio, Texas, USA. Neither of these disclosures meets the terms of the appended claims and both represent the results of scientific investigations made without reference to the development of a practical ophthalmological surgical tool.

EP—A—0111060, which was published after the claimed priority date of the present application but itself claims an earlier priority date and thus belongs to the state of the art under the provisions of Article 54(3) of the European Patent Convention, discloses and claims an ablative photodecomposition apparatus for photoetching a biological layer comprised of an organic material by means of ultraviolet radiation focussed upon said layer. However, EP—A—0111060 contains no disclosure of a developed sculpture apparatus for performing ophthalmological surgery such as is disclosed and claimed in the present application. For example, whilst EP—A—0111060 makes mention of scanning of the laser beam, there is no disclosure in EP—A—0111060 of the microprocessor controlled area scanning that is performed in operation of the apparatus of the present invention.

US—A—3769963 discloses an instrument for performing microsurgery and there is disclosure of the use of laser radiation in the ultraviolet range, but as with EP—A—0111060 there is no disclosure or suggestion in US—A—3769963 of a developed ophthalmological corneal sculpture apparatus according to the teachings of the present invention.

DE—A—3148748 discloses an apparatus for performing radial keratotomy operations by use of a so-called laser scalpel subject to microcomputer control. There is no disclosure in DE—A—3148748 of the use of ultraviolet radiation and indeed, whereas the present invention is concerned with ablative photodecomposition of corneal tissue in the optically active region of the cornea which necessitates the use of laser radiation at wavelengths such as to avoid scar formations, the whole purpose of the apparatus disclosed in DE—A—3148748 is to achieve scar formation outside of the optically active region of the cornea. The disclosure of DE—A—3148748 is thus the antithesis of the present invention and does not meet the terms of the claims appended hereto.

Further features of the present invention are set forth with particularity in the appended claims and will be described in detail hereinafter in order that the present invention and the ways in which it is distinguished from the prior art might be well understood. Referring to the accompanying drawings:

Fig. 1 is a schematic diagram in perspective, to show the general arrangement of operative components of an embodiment of the invention;

Fig. 2 is a simplified view in longitudinal section, showing an eye-retaining fixture used with the apparatus of Fig. 1;

Figs. 3 and 4 are simplified diagrams to illustrate different scan patterns performed with apparatus as in Fig. 1;

Figs. 5 and 6 are simplified sectional views to illustrate different sculptured surface curvatures achieved with either of the scan patterns of Figs. 3 and 4;

Figs. 7 and 8 are views in section, and Fig. 9 is a view in front elevation, to illustrate use of the invention in a corneal transplant operation; and

Figs. 10 and 11 are, respectively, a view in front elevation and an enlarged half-section-profile diagram to illustrate a Fresnel-cut use of the invention.

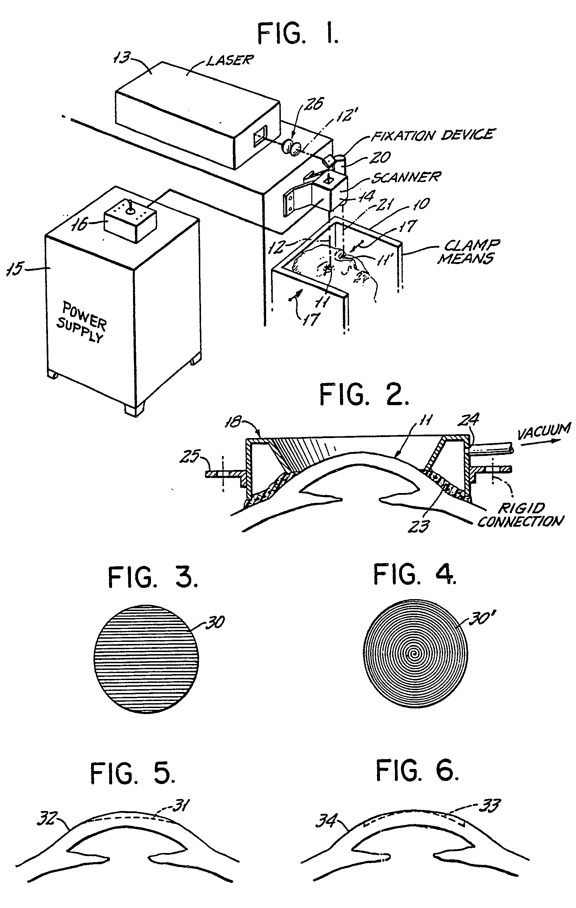

In Fig. 1, clamp means 10 is shown for fixed retention of the head of a patient (reclined, face up) such that the eye 11 to be operated upon is fixedly aligned with a downwardly folded portion 12 of the central axis 12' of beam output from a stationary laser device 13, and scanner means 14 is provided for programmed deflection of laser-beam output, with respect to the central axis 12. The laser device 13 is served by a suitable power supply 15, and the scanner means 14 includes selectively operable control means, symbolized at 16, for determining scan pattern, effective limits of scan action, and, if desired, the time-varying profile of one or more dimensional components of scan action.

Preferably, the clamp means 10 includes means, symbolized at 17, to stabilize the patient's head via opposed engagements at the region of his temples, and an eye-retaining fixture (18, Fig. 2) peripherally engages eye 11 at the corneal-scleral area. Also preferably, an optical-fixation device 20 is adjustably, fixed, as to the housing of scanner 14. Illustratively, device 20 includes a sighting reticle and lens, whereby the eye 11' not being operated upon can view the reticle as if at infinity; the sighting alignment 21 for device 20 is parallel to the axis 12, and it will be understood that adjustable means (not shown) may provide an adjustable offset, as needed for accommodation of the patient's interpupilary distance and to adapt to the particular mounted offset of device 20 from axis 12. For an operation on the other eye 11', the eye 11 will be available for similar fixation, in conjunction with another fixation device (not shown) and associated adjustable offsetting means; alternatively, the fixation device 20 may be adjustably mounted at correct offset on the opposite side of scanner 14. For purposes of operating on eye 11', clamp means 10 will have been indexed laterally with respect to laser 13 to the extent aligning axis 12 with the eye (11') then to be operated upon, thereby positioning eye 11 for use of the fixation device.

The eye-retaining fixture 18 of Fig. 2 is seen to comprise a hollow annulus, having a convergent axial-end wall 23 of air-permeable material contoured to engage and retain the eye via a scleral-corneal region. A side-port connection 24 to a vacuum pump enables retention of eye engagement to wall 23, and outward lug or flange means 25 enables rigid aligned and spaced connection of

fixture 18 to laser 13 and its scanner 14 via means suggested by legend in Fig. 2, such means being omitted from Fig. 1 for reasons of more simplified showing.

The laser selected for use at 13 emits in the ultraviolet, namely, at wavelengths of less than substantially 400 nanometers. Such emissions for gas lasers are characteristically at 351 nm for xenon-fluoride lasers, 337 nm for nitrogen lasers, 308 nm for xenon-chloride lasers, 248 nm for krypton-fluoride lasers, 193 nm for argon fluoride lasers, and 157 nm for fluorine lasers; and within this range, frequency-doubling techniques applied to other lasers, including crystal lasers, provide further alternative sources.

One of the existing commercial excimer-laser products of Lambda Physik GmbH, Göttingen, Germany, for example their Model EMG 103 operating with argon-fluoride, is satisfactory for use as laser 13; for this product, maximum energy per pulse is 200 millijoules, with a pulse-repetition rate of 200 per second, $3 \times 10^5$ shots being available from a single charge of the involved gas, before reducing to 50 percent of specified power at this repetition rate, it being noted that full rated power is not necessarily required in use of the present invention. Pulse width is about 15 nanoseconds, and typical beam dimensions at 25 centimeters (10 inches) are 10 mm×22 mm. To bring this down to an illustratively useful rounded-square spot size of 0.5 mm by 0.5 mm at the eye 11, corrective lens elements at 26, as of quartz, calcium fluoride, or magnesium fluoride, will be understood to include a cylindrical element and a spherical element whereby beam size is reduced while the rectangular section is compressed to substantially square section.

The requirements for the laser are that beam exposure flux should be adjustable to a level at which tissue ablation per scan is to an ascertained elemental depth which is but a fraction of the desired maximum depth of ablation into the stroma region of the eye. For a pulsed laser, the tissue penetration per pulse must be but a fraction the desired maximum ablation depth.

Figs. 3 and 4 illustrate alternative scan patterns for having the typical half-millimeter focused and repetitively pulsed spot of the laser beam course the surface of eye 11 in the performance of a surgical procedure. The circle 30 in Fig. 3, may illustratively be of 6 mm diameter at the cornea, and centered on the axis of eye 11. The scan action is rectilineal, involving plural horizontal line scans with progressive vertical displacement to cover the field, here shown limited to the circle 30. For this purpose, a suitable scanner, known as "Microscan 771", is commercially available from Laser Industries International, Hendon, England and therefore need not be here described in detail. It suffices to say that the scanner comprises mechanically displaceable optical components and control means 16 associated with such components for displacing them and including a microprocessor with memory for delineated boundary limits of scan, such as the limiting circle 30. The delineation can be to the surgeon's desired boundary contours, and the scan speed and direction may be programmed or manually controlled. What has been said as to Fig. 3 also applies to Fig. 4, except that a spiral course of scan, i.e., rotary sweeps at progressively changing radius, is involved in each coverage of the delineated field 30'.

It is a feature of the invention that the programming of scan action be such that predetermined depth of ultraviolet laser incision be made to effectively recharacterize the external contour of the cornea within the entire predetermined field boundary (e.g., 30, 30'). This is done by progressive precise photodecomposition of the corneal tissue, as to a depth limit of 0.35 mm. In the illustrative argon-fluoride laser referenced above, a precise volume of tissue (e.g., 14 microns deep) may be excised for each laser pulse or shot, and the half-millimeter spot, repeated at 200/second, can cover the entire area within the delineated boundary 30, in about fifteen seconds.

For the situation depicted in Fig. 5, the dashed line 31 represents the ultimate curvature to which the external surface of a cornea 32 may be modified to achieve a change in optical properties of the involved eye, here illustratively a myopic eye, for which the reduced curvature 31 offers a diopter-reducing corrective effect, all without resort to the use of a spectacle lens or a contact lens to achieve the result. To achieve the curve 31, the minimum desired photocomposition is at the outer boundary 30, and the maximum is at the center. This is achievable by programming the microprocessor to progressively reduce the radius of the boundary circle 30 (i.e., progressively reduce the area of scanned field), for successive scans of the reducing field. If the curvature 31 requires a maximum depth of 0.35 mm of cornea removal at the center, this means that the central region of the cornea (i.e., the last and most reduced scanned field) will have been scanned twenty-five times, and that cornea removal outside this most reduced scanned field will have involved lesser numbers of scans, the progression having been predetermined to achieve the desired ultimate curvature 30 over the area 31.

What has been said as to the scan technique of Fig. 3 to achieve curvature 31 applies equally for use of the spiral scan of Fig. 4, the field 30' again being programmed for automatic reduction as necessary to provide maximun cornea removal at the center, and minimum at outer limits of the circular boundary.

What has been said as to programming to achieve lesser curvature in the outer surface of the cornea (Fig. 5), to reduce a myopic condition, applies also to Fig. 6 for reduction of a hyperopic condition. In Fig. 6, the difference lies in programming field scans so as to initiate and progressively enlarge a central area which defines the inner limit of field scanned. Thus, except for perhaps one field scan involving cornea removal over the entire area bounded by circle 30 (30'), all remaining field-scanned areas are annular, with progressively increasing inner radius of each successively scanned annular field. The last such "field" will necessarily be virtually a circular line at the diameter of circle 30 (30'), along which circular line the depth of surgical excision will have been greatest, as indicated by dashed line 33 in the cornea 34 of Fig. 6.

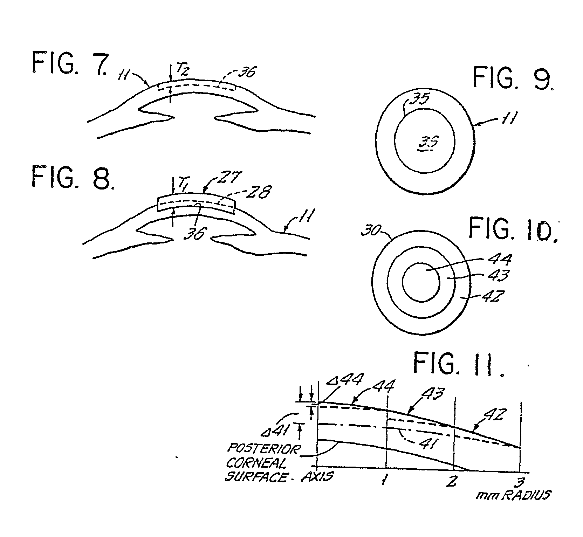

Quite aside from the variable-depth character of the removal of corneal tissue (Figs. 5 and 6), the invention also lends itself to uniform-depth removals, over the entire area of a multiply-scanned constant field. In Figs. 7 and 9, the cornea of an eye 11 is subjected to a succession of scans of (i.e., within) a constant predetermined field area 35. In the illustrative laser case, with excision to a depth of 14 microns for each pulse, a uniform depth of 0.35 mm is achieved by 25 scans of the total area 35, to produce a carved base or floor curvature 36 for reception and location of a corneal transplant.

Further with respect to a corneal-transplant procedure, the described apparatus will be seen to be further useful, as in preparation of the corneal insert to be implanted at and within the recess 36. A donated eye may be reversibly held to a fixture as described at 18 in Fig. 2; by "reversible" it is meant that, depending upon the manner of mounting flange 25, either the epithelium or the endothelium of the donated eye may be mounted for upward exposure to the laser beam 12, it being understood that for the latter situation with the donated eye, iris and other regions not needed for corneal-scleral mounting and for corneal operation will have been initially removed. A preferred procedure is first to so expose to laser scanning the concave inner side of the donated cornea; such scanning is to an extent (achieved by multiple scans of a full circular field exceeding the diameter of recess 36) sufficient to remove tissue at least to a uniform depth within the stroma, whereupon the mounting of fixture 18 (and its partially machined corneal workpiece) is reversed, to expose to laser scanning the convex outer side of the donated cornea. Scanning the outer side consists of two steps; first, multiple scans of the full circular field (exceeding the diameter of recess 36), thereby excising at least the epithelium and to a depth which preferably achieves a transplant thickness $T_1$ exceeding the depth $T_2$ of recess 36; second, scanner 14 is operated in a line-cutting mode wherein successive laser pulses sequentially advance along the circumference of a circle designed for precise acceptance in the circular recess 36, until full severance of the circular cutout, which then becomes the prepared transplant. Upon implanting, donated stroma is placed in full endothelium-free contact with the patient's prepared stroma, and the implant may be sutured. Later, upon removal of sutures, the outer surface of the eye 11 and its transplant 27 will have the appearance shown in Fig. 8, wherein the transplant projects beyond adjacent areas of the patient's cornea, and this projecting surface of the transplant may be reduced by laser scanning to a finish contour 28 of preferably flush marginal conformance with non-sculptured adjacent tissue of the patient's eye. It will be further understood that, subject to the surgeon's decision, such a finishing cut may be to a curvature which does or does not effect a predetermined change in optical performance of the eye.

Certain myopic and hyperopic conditions may be so severe that to produce merely an excised single surface 31 or 33 could involve, in the surgeon's considered judgement, an excessive removal of tissue, at the involved region of necessarily deepest cut. For such a situation, the invention offers the option of programming successive scans in a manner to create a Fresnel-type stepped development of the desired ultimate curvature. Such a situation and procedure are illustrated in Figs. 10 and 11, wherein an ultimately reduced-curvature surface 31 of Fig. 5 (dashed line 41 in Fig. 11) is achieved in annular increments within the field area bounded at 30. In the outer one of these annuli 42, the curvature and depth of cut are precisely as would have applied to generate the continuous curve 41 (i.e., without Fresnel steps). But the intermediate annular area 43 effectively achieves a continuation of curve 41 with much less volume of corneal excision. Finally, the inner circular area 44 effectively completes curve 41, with minimal removal of corneal tissue.

The removal of tissue at the center is denoted $\Delta_{44}$ for the Fresnel cut 44 of Figs. 10 and 11 and, comparatively, is but a small fraction of the maximum removal depth $\Delta_{41}$ which would be needed to achieve the same optical correction with the smoothly developed corrected single-curvature surface 41. It will be understood that for a Fresnel-type cut as illustrated in Fig. 11, the previously described illustrative half-millimeter spot size will be incapable of achieving the desired result, for the one-millimeter radial increments shown in Fig. 11. To produce the requisite resolution for characterizing increments of curvature 41 at 42, 43, 44, it is necessary to employ a small spot size. For the indicated Lambda Physik equipment, spot-size reduction is feasible via means 26 as far as to produce a 30-micron spot size, if necessary; with this capability, it is seen that the one-millimeter radius increments of annulus 42 and annulus 43 are each achievable with a resolution of about 35 radial steps per increment (42 or 43). It will thus be understood that numbers given above are for purposes of more simplified illustration of this and the other aspects of the present invention.

Scanner action may further be achieved by the deflection of the laser beam selectively in orthogonal X and Y directions having an adjustable angular orientation relative to the eye to be treated. With suitable means indicating the currently operative extent of angular adjustment, orthogonally related deflection signals can be applied to suitably synchronized X-axis and Y-axis deflectors for rectilinearly scanned deflection of the laser beam at the eye within limits established by envelope-limiting means associated with the deflection-signal generators and co-ordinating with enable/disable functions of the laser whereby the laser beam is only operatively scanned over the desired limited area of the cornea. It is a feature of the invention that by suitably programming the rate of scan of one of these scan axes, for example, the X or line-scan deflection axis, the rate can be relatively slow at outer limits and relatively fast in regions between outer limits of each scan, with the result that the greater density of pulsed-laser shots will impact the latter regions, while the Y-axis component of scan development remains steady; alternatively, the Y-axis component may be under a varying rate control while the X-axis sweeps are linear. The result is to develop an astigmatic curvature of cylindrical nature, which may be oriented according to the preset angular direction. The prospect is thus to effect astigmatic correction in a given eye, without resort to an artificial element such as a lens.

A polar-co-ordinate scan pattern or the spiral-type pattern referred to in connection with Fig. 4, could alternatively be developed by use of a single radial-deflection beam deflection means served by a radius-scan sweep-signal generator, and with rotary sweep obtained by selectively orienting the beam deflection means. A polar raster generator co-ordinates the sweep orientation with sweep cycles of the sweep-signal generator. The specific spiral scan of Fig. 4 can be achieved in this way by making the radial sweep cycle of the sweep-signal generator so slow with respect to the rotary sweep that plural rotations are involved for each radial sweep. On the other hand, with a slow rotary sweep and many radial sweeps for each such rotation, a polar scan can be developed for the delineated field. If the signal output of the sweep-signal generator is sufficient to cause a full diameter of sweep for each sweep cycle, the central region of the field will receive the greatest density of shots and will therefore be subjected to maximum excision; the field will therefore be covered once for each half revolution of rotary sweep. The result of such shot-density distribution is to approach the requirement for curvature reduction, as discussed in connection with Fig. 6, and it will be understood that rate-profiling control of the radius-scan sweep-signals offers further latitude in the development of ultimate curvature upon completion of scanning.

## Claims

1. Sculpture apparatus for performing ophthalmological surgery on the optically used portion of the anterior surface of the cornea, said apparatus comprising:

laser means (13) adapted for producing an output beam having a cross-section that at corneal impingement is small in relation to said optically used portions, said output beam being in the ultraviolet portion of the electromagnetic spectrum and being capable of achieving selective ablation of said anterior surface with penetration into the stroma to achieve a volumetric removal of corneal tissue by photodecomposition,

laser control means (16), including a microprocessor, for controlling the operation of said laser means (13) so as to effect a predetermined operative procedure, said laser control means including means (14) for scan deflection of said beam for selectively impinging the beam throughout a predetermined area within said optically used portion, the irradiated flux density and exposure time of laser-beam impingement at the cornea being such as to achieve for each scan an ablation depth which is but a fraction of the maximum depth of a required penetration; and

said laser control means (16) being adapted, at least in part by the programming of said microprocessor, to effect successive scans within said predetermined area, whereby to achieve said required penetration as a result of said successive scans.

2. Apparatus according to claim 1 wherein said laser means (13) is an excimer laser operative with a gas comprising fluorine, argon fluoride, krypton fluoride, xenon chloride or xenon fluoride.

3. Apparatus according to claim 1 or 2 wherein said laser means (13) is adapted to produce an output beam having a wavelength not substantially exceeding 400 nm.

4. Apparatus according to any preceding claim comprising positioning means (10, 18, 20) for determining the position of an eye to be operated upon relative to said laser means, and wherein said positioning means includes optical-fixation means (20) fixed with respect to said laser means (13) and aligned for observation through the other eye of the patient.

5. Apparatus according to any preceding claim wherein eye-fixation means (18) is provided for steadying the cornea of the eye which is to be operated upon with respect to said laser means (13) and with the central area of the cornea centred on the central axis of scan deflection of the laser output beam, said eye-fixation means (18) including a circumferentially continuous hollow annular ring (23) which is air permeable on one axial side, said side being contoured for adaptation to the corneal scleral region of an eye, and an external connection port (24) communicating with the hollow of said ring for external air-evacuating connection of the same.

6. Apparatus according to any preceding claim wherein said laser means (13) includes means (26) for reducing the beam cross-section at the eye of the patient to a spot size in the range of 30 microns to 0.5 mm.

7. Apparatus according to any preceding claim wherein said means (14) for scan deflection of said laser beam comprises mechanically displaceable optical components and means for displacing said optical components to effect a predetermined deflection of said beam.

8. Apparatus according to any preceding claim wherein said laser means (13) includes means for adjusting beam-exposure flux to a level at which resultant corneal-tissue ablation scan is to an ascertained elemental depth which is but a fraction of the desired maximum depth of ablation into the stroma region of the eye.

9. Apparatus according to claim 8 wherein said laser output beam is a pulsed beam, and said adjusting means is adapted for setting the beam intensity to produce tissue penetration to a depth per pulsed exposure which is but a fraction of said desired maximum depth.

10. Apparatus according to any preceding claim wherein said means (14) for scan deflection of said laser beam provides for controlled beam deflection transverse to the beam propagation direction for enabling beam impingement area coverage of an optically functioning area of the cornea, and said laser control means (16) is adapted to establish a plurality of different perimeter limits of area-scan action within the perimeter of said optically functioning area of the cornea and to co-ordinate the operation of said means (14) for scan deflection of said laser beam in a controlled program of limitation of one area scan within one perimeter limit before repeating such co-ordination within the next successive perimeter limit, whereby ablative penetration to the desired depth is the cumulative result of plural area scans of each of a succession of different but overlapping areas.

11. Apparatus according to claim 10 wherein said means (14) for scan deflection of said laser beam is radially operative with respect to the axis of said beam at incidence with the cornea and includes means for rotating the direction in which the radial deflection is operative whereby, in the course of a given scanning operation, each area scan is the result of a spirally developed course of beam deflection.

12. Apparatus according to claim 10 or 11 in which said perimeter limits are circular outer limits of successively reducing different concentrically related scanned circular areas, whereby the cumulative result of successive area scanning of the cornea is myopia correcting.

13. Apparatus according to claim 10 or 11 in which said perimeter limits are circular limits of different concentrically related scanned annular areas of constant outer diameter and successively increasing inner diameter, whereby the cumulative result of successive area scanning of the cornea is hyperopia-correcting.

14. Apparatus according to any of claims 1 to 9 in which the perimeter limit of successive area scanning is a circle of constant radius, whereby the cumulative result of successive area scanning of the cornea is to prepare a circular corneal recess of constant depth for reception of a corneal transplant.

15. Apparatus according to any of claims 1 to 13 wherein said laser control means (16) is arranged such that successive area scans create a Fresnel-type stepped development of the desired ultimate curvature.

16. Apparatus according to claim 15 wherein said laser control means (16) is arranged to control the operation of said means (14) for scan deflection of the laser beam in each of a plurality of concentrically related adjacent annular zonal areas, and said laser control means (16) is further arranged for successive area scanning of each annular zonal area in a pattern of outer perimeter radius variation at constant inner perimeter radius, the apparatus thereby being arranged for producing a Fresnel-characterized myopia-correcting anterior corneal surface profile.

17. Apparatus according to claim 15 wherein said laser control means (16) is arranged to control the operation of said means (14) for scan deflection of the laser beam in each of a plurality of concentrically related adjacent annular zonal areas, and said laser control means (16) is further arranged for successive area scanning of each annular zonal area in a pattern of inner perimeter radius variation at constant outer perimeter radius, the apparatus thereby being arranged for producing a Fresnel-characterized hyperopia-correcting anterior corneal surface profile.

18. Apparatus according to any of claims 1 to 9 wherein said means (14) for scan deflection of the laser beam is adapted to provide two orthogonal components of controlled beam deflection transverse to the beam propagation direction, and said laser control means (16) is adapted for co-ordinating the operation of said means (14) for scan deflection of the laser beam in a controlled program of area coverage to establish greatest cumulative beam exposure along the alignment of the central axis of symmetry of an ascertained astigmatic condition, with cumulative beam exposure decreasing as a function of increasing lateral offset on both sides of said central axis of symmetry, the apparatus thereby being adapted for correction of an astigmatic condition.

19. Apparatus according to claim 18 in which cumulative beam exposure is varied by varying the scan rate of said laser output beam scanning means.

**Patentansprüche**

1. Skulptur-Vorrichtung zur Durchführung einer ophtalmologischen Chirugie an dem optisch genutzten Bereich der äußeren Oberfläche der Kornea, mit:

einer Laser-Vorrichtung (13), die ein Ausgangsstrahlenbündel erzeugen kann, dessen Querschnitt bei Auftreffen auf die Kornea klein im Vergleich zu dem optisch genutzten Bereich ist, wobei das Ausgangsstrahlenbündel im ultravioletten Bereich des elektromagnetischen Spektrums liegt und eine wahlweise Abtragung der äußeren Oberfläche unter Eindringung in das Stroma zur Erzielung eines volumetrischen Entfernens von Kornea-Gewebe mittels Photozersetzung bewirken kann, und

einer Laser-Steuervorrichtung (16), die einen Mikroprozessor aufweist, um den Betrieb der Laser-Vorrichtung (13) derart zu steuern, daß eine gewünschte Arbeitsweise erreicht wird, wobei die Laser-Steuervorrichtung eine Vorrichtung (14) zur Abtast-Ablenkung des Strahlenbündels zum wahlweisen Auftreffen des Strahlenbündels überall in einem vorbestimmten Abschnitt innerhalb des optisch genutzten Bereichs umfaßt, wobei die Bestrahlungsflußdichte und die Belichtungszeit der Laserstrahl-Einwirkung an der Kornea derart festgelegt sind, daß für jede Abtastung eine Abtragungstiefe erzielt wird, die nur ein Bruchteil der maximalen Tiefe der notwendigen Eindringung ist,

wobei die Laser-Steuervorrichtung (16) in der Lage ist, zumindest teilweise aufgrund der Programmierung des Mikroprozessors innerhalb des vorbestimmten Abschnitts aufeinanderfolgende Abtastungen zu bewirken, um dadurch die notwendige-Eindringung als Folge der aufeinanderfolgenden Abtastungen zu erreichen.

2. Vorrichtung nach Anspruch 1, wobei die Laser-Vorrichtung (13) ein Excimer-Laser ist, der mit einem Gas arbeitet, das Fluor, Argonfluorid, Kryptonfluorid, Xenonchlorid oder Xenonfluorid enthält.

3. Vorrichtung nach Anspruch 1 oder 2, wobei die Laser-Vorrichtung (13) in der Lage ist, ein Ausgangsstrahlenbündel mit einer Wellenlänge im wesentlichen nicht über 400 nm zu erzeugen.

4. Vorrichtung nach einem der vorhergehenden Ansprüche, mit einer Positioniervorrichtung (10, 18, 20) zur Festlegung der Position eines zu operienden Auges relativ zu der Laser-Vorrichtung, wobei die Positioniervorrichtung eine optische Fixiervorrichtung (20) aufweist, die bezüglich der Laser-Vorrichtung (13) fest ist und für die Beobachtung durch das andere Auge des Patienten ausgerichtet ist.

5. Vorrichtung nach einem der vorhergehenden Ansprüche, wobei eine Augen-Festlegungsvorrichtung (18) zur Festlegung der Kornea des zu operierenden Auges bezüglich der Laser-Vorrichtung vorgesehen ist und der zentrale Bereich der Kornea auf der Mittelachse der Abtast-Ablenkung des Laser-Ausgangsstrahlenbündels zentriert ist, wobei die Augen-Festlegungsvorrichtung (18) einen in Umfangsrichtung kontinuierlichen, hohlen kreisförmigen Ring (23), der an einer axialen Seite, die zur Anpassung an den Kornea-Sklera-Bereich eines Auges konturiert ist, luftdurchlässig ist, und eine externe Verbindungsöffnung (24) aufweist, die mit dem Hohlraum des Ringes zwecks dessen externer Luft-Entleerung in Verbindung steht.

6. Vorrichtung nach einem der vorhergehenden Ansprüche, wobei die Laser-Vorrichtung (13) eine Vorrichtung (26) zur Verringerung des Strahlenbündel-Querschnitts an dem Auge des Patienten auf eine Punktgröße im Bereich von 30 Mikrometer bis 0,5 mm aufweist.

7. Vorrichtung nach einem der vorhergehenden Ansprüche, wobei die Vorrichtung (14) zur Abtast-Ablenkung des Laser-Strahlenbündels mechanisch verstellbare optische Komponenten und eine Vorrichtung zur Verstellung der optischen Komponenten aufweist, um eine vorbestimmte Ablenkung des Strahlenbündels zu erzielen.

8. Vorrichtung nach einem der vorhergehenden Ansprüche, wobei die Laser-Vorrichtung (13) eine Vorrichtung zur Einstellung des Strahlenbündel-Belichtungsflusses auf einen Wert aufweist, bei dem die sich ergebende Korneagewebe-Abtragungsabtastung einer festgestellten elementaren Tiefe entspricht, die nur einen Bruchteil der gewünschten maximalen Tiefe der Abtragung in dem Stroma-Bereich des Auges beträgt.

9. Vorrichtung nach Anspruch 8, wobei das Laser-Ausgangsstrahlenbündel ein Impuls-Strahlenbündel ist und wobei die Einstellvorrichtung in der Lage ist, die Strahlenbündel-Intensität so festzusetzen, daß eine Gewebe-Eindringung pro Impuls-Belichtung mit einer Tiefe erreicht wird, die nur ein Bruchteil der gewünschten maximalen Tiefe ist.

10. Vorrichtung nach einem der vorhergehenden Ansprüche, wobei die Vorrichtung (14) zur Abtast-Ablenkung des Laser-Strahlenbündels eine gesteuerte Strahlenbündel-Ablenkung quer zur Strahlenbündel-Ausbreitungsrichtung bewirkt, um eine Überdeckung des Strahlenbündel-Auftreffbereichs mit einem optisch wirksamen Bereich der Kornea zu ermöglichen, und wobei die Laser-Steuervorrichtung (16) in der Lage ist, eine Vielzahl von unterschiedlichen Perimeter-Grenzen der Bereichsabtastung innerhalb des Perimeters des optisch wirksamen Bereichs der Kornea festzusetzen und den Betrieb der Vorrichtung (14) zur Abtast-Ablenkung des Laser-Strahlenbündels in einem gesteuerten Programm der Begrenzung einer Bereichsabtastung innerhalb einer Perimeter-Grenze zu koodinieren, bevor eine derartige Koordinierung innerhalb der nächstfolgenden Perimeter-Grenze wiederholt wird, wodurch die abtragende Eindringung bis zur gewünschten Tiefe das summierte Ergebnis mehrerer Bereichsabtastungen von jeweils einer Folge von unterschiedlichen, aber sich überlappenden Bereichen ist.

11. Vorrichtung nach Anspruch 10, wobei die Vorrichtung (14) zur Abtast-Ablenkung des Laser-Strahlenbündels bezüglich der Achse des Strahlenbündels beim Auftreffen auf die Kornea radial arbeitet und eine Vorrichtung zur Drehung der Richtung aufweist, in der die radiale Ablenkung durchgeführt wird, wodurch in der Bahn eines vorgegebenen Abtastvorgangs jede Bereichsabtastung das Resultat einer sich spiralförmig entwickelnden Bahn der Strahlenbündel-Ablenkung ist.

12. Vorrichtung nach Anspruch 10 oder 11, wobei die Perimeter-Grenzen kreisförmige äußere Grenzen von aufeinanderfolgenden, reduzierten, unterschiedlichen, konzentrisch angeordneten, abgetasteten, kresiförmigen Bereichen gebildet sind, wodurch das summierte Resultat von aufeinanderfolgenden Bereichsabtastungen der Kornea myopie-korrigierend ist.

13. Vorrichtung nach Anspruch 10 oder 11, wobei die Perimeter-Grenzen kreisförmige Grenzen von unterschiedlichen, konzentrisch angeordneten, abgetasteten, ringförmigen Bereiche mit konstantem äußeren Durchmesser und allmählich anwachsendem inneren Durchmesser sind, wodurch das summierte Resultat von aufeinanderfolgenden Bereichsabtastungen der Kornea hyperopie-korrigierend ist.

14. Vorrichtung nach einem der Ansprüche 1 bis 9, wobei die Perimeter-Grenze aufeinanderfolgender Bereichsabtastungen ein Kreis mit konstantem Radius ist, wodurch das summierte Resultat von aufeinanderfolgenden Bereichsabtastungen der Kornea der Ausbildung einer kreisförmigen Kornea-Ausnehmung konstanter Tiefe zur Aufnahme eines Kornea-Transplantats dient.

15. Vorrichtung nach einem der Ansprüche 1 bis 13, wobei die Laser-Steuervorrichtung (16) derart angeordnet ist, daß aufeinanderfolgende Bereichsabtastungen eine Fresnel-artige, abgestufte Entwicklung der gewünschten endgültigen Krümmung erzeugen.

16. Vorrichtung nach Anspruch 15, wobei die Laser-Steuervorrichtung (16) derart ausgebildet ist, daß sie den Betrieb der Vorrichtung (14) zur Abtast-Ablenkung des Laser-Strahlenbündels in jeder einer Veilzahl von konzentrisch angeordneten, benachbarten ringförmigen Zonenbereichen steuert, und wobei die Laser-Steuervorrichtung (16) weiterhin derart ausgebildet ist, daß sie die aufeinanderfolgenden Bereichsabtastungen jedes ringförmigen Zonenbereichs mit einem Muster der Variation des äußeren Perimeter-Radius bei konstantem inneren Perimeter-Radius durchführt, wodurch die Vorrichtung ein Fresnelartiges, myopie-korrigierendes äußeres Kornea-Oberflächenprofil ausbildet.

17. Vorrichtung nach Anspruch 15, wobei die Laser-Steuervorrichtung (16) derart ausgebildet ist, daß sie den Betrieb der Vorrichtung (14) zur Abtast-Ablenkung des Laser-Strahlenbündels in jeder einer Vielzahl von konzentrisch angeordneten, benachbarten ringförmigen Zonenbereichen steuert, und wobei die Laser-Steuervorrichtung (16) weiterhin derart ausgebildet ist, daß sie die aufeinanderfolgenden Bereichsabtastungen jedes ringförmigen Zonenbereichs mit einem Muster der Variation des inneren Perimeter-Radius bei konstantem äußeren Perimeter-Radius durchführt, wodurch die Vorrichtung ein Fresnelartiges, hyperopie-korrigierendes äußeres Kornea-Oberflächenprofil ausbildet.

18. Vorrichtung nach einem der Ansprüche 1 bis 9, wobei die Vorrichtung (14) zur Abtast-Ablenkung des Laser-Strahlenbündels zwei orthogonale Komponenten der gesteuerten Strahlenbündel Ablenkung quer zur Strahlenbündel-Ausbreitungsrichtung bilden kann und wobei die Laser-Steuervorrichtung (16) den Betrieb der Vorrichtung (14) zur Abtast-Ablenkung des Laser-Strahlenbündels in einem gesteuerten Programm von Bereichsüberdeckungen koordinieren kann, um die größte summierte Strahlenbündel-Belichtung entlang der Ausrichtung der Symmetrie-Mittelasche eines festgestellten astigmatischen Zustandes zu erzeugen, wobei die summierte Strahlenbündel-Belichtung als Funktion des Anwachsen des seitlichen Versatzes zu beiden Seiten der Symmetrie-Mittelachse abnimmt, wodurch die Vorrichtung die Korrektion eines astigmatischen Zustandes bewirken kann.

19. Vorrichtung nach Anspruch 18, wobei die summierte Strahlenbündel Belichtung mittels der Veränderung der Abtastrate der Laser-Ausgangsstrahlenbündel-Abtastvorrichtung verandert wird.

## Revendications

1. Appareil de sculpture pour réaliser des opérations de chirurgie ophtalmologique sur la partie utilisée optiquement de la surface antérieure de la cornée, ledit appareil comprenant:

un moyen laser (13), apte à produire un faisceau de sortie ayant une section transversale qui, au niveau de l'impact sur la cornée, est faible par rapport à ladite partie utilisée optiquement, ledit faisceau de sortie se trouvant dans la région des ultraviolets du spectre électromagnétique et étant apte à réaliser une ablation sélective de ladite surface antérieure avec pénétration dans le stroma pour réaliser un retrait volumétrique de tissu cornéen par photodécomposition;

un moyen de commande de laser (16), comprenant un microprocesseur, pour commander le fonctionnement dudit moyen laser (13), de façon à effectuer un processus opératoire prédéterminé, ledit moyen de commande de laser comprenant un moyen (14) de déflexion de balayage dudit faisceau afin de permettre un impact sélectif de celui-ci sur une zone prédéterminée à l'intérieur de ladite partie utilisée optiquement, la densité de flux rayonné et le temps d'exposition de l'impact du faisceau laser au niveau de la cornée étant tels que l'on obtienne, pour chaque balayagé, une profondeur d'ablation qui n'est qu'une fraction de la profondeur maximale d'une pénétration requise; et

ledit moyen de commande de laser (16) étant apte, au moins en partie par la programmation dudit microprocesseur, à effectuer des balayages successifs à l'intérieur de ladite zone prédéterminée, permettant ainsi de réaliser ladite pénétration requise par suite desdits balayages successifs.

2. Appareil selon la revendication 1, dans lequel ledit moyen laser (13) est un laser excimer opérant avec un gaz comprenant du fluor, du fluorure d'argon, du fluorure de krypton, du chlorure de xénon ou du fluorure de xénon.

3. Appareil selon la revendication 1 ou 2, dans lequel ledit moyen laser (13) est apte à produire un faisceau de sortie ayant une longueur d'onde ne dépassant pas sensiblement 400 nm.

4. Appareil selon l'une quelconque des revendications précédentes, comprenant un moyen de positionnement (10, 18, 20) pour déterminer la position, par rapport audit moyen laser, d'un oeil sur lequel on doit opérer, et dans lequel ledit moyen de positionnement comprend un moyen de fixation optique (20) fixe par rapport audit moyen laser (13) et orienté en vue d'une observation à travers l'autre oeil du patient.

5. Appareil selon l'une quelconque des revendications précédentes, dans lequel un moyen de fixation (18) de l'oeil est prévu pour stabiliser la cornée de l'oeil, sur lequel on doit opérer, par rapport audit moyen laser (13) et avec la zone centrale de la cornée centrée sur l'axe central de déflexion de balayage du faisceau de sortie du laser, ledit moyen de fixation (18) de l'oeil comprenant une bague annulaire (23), creuse et continue sur son pourtour, qui est perméable à l'air sur l'une de ses faces axiales, ladite face présentant un contour adapté à la région sclérale cornéenne d'un oeil, et un orifice de raccordement externe (24) communiquant avec la partie creuse de ladite bague en vue de son raccordement à une évacuation d'air externe de cette dernière.

6. Appareil selon l'une quelconque des revendications précédentes, dans lequel ledit moyen laser (13) comprend un moyen (26) pour réduire la section transversale du faisceau au niveau de l'oeil du patient à une dimension de spot se situant dans la plage de 30 microns à 0,5 mm.

7. Appareil selon l'une quelconque des revendications précédentes, dans lequel ledit moyen (14) de déflexion de balayage dudit faisceau laser comprend des composants optiques déplaçables mécaniquement et un moyen pour déplacer lesdits composants optiques afin d'effectuer une déflexion prédéterminée dudit faisceau.

8. Appareil selon l'une quelconque des revendications précédentes, dans lequel ledit moyen laser (13) comprend un moyen pour ajuster le flux d'exposition du faisceau à un niveau auquel le balayage d'ablation du tissu cornéen résultant se trouve à une profondeur élémentaire déterminée qui n'est qu'une fraction de la profondeur maximale désirée d'ablation dans la région du stroma de l'oeil.

9. Appareil selon la revendication 8, dans lequel ledit faisceau de sortie du laser est un faisceau pulsé, et ledit moyen d'ajustement est apte à régler l'intensité du faisceau pour obtenir une pénétration du tissue à une profondeur, par exposition d'impulsion, qui n'est qu'une fraction de ladite profondeur maximale désirée.

10. Appareil selon l'une quelconque des revendications précédentes, dans lequel ledit moyen (14) de déflexion de balayage dudit faisceau laser fournit une déflexion contrôlée du faisceau transversale à la direction de propagation du faisceau pour permettre une couverture de la zone d'impact du faisceau d'une zone optiquement active de la cornée, et ledit moyen de commande de laser (16) est apte à établir une pluralité de différentes limites de périmètre d'action de balayage de zone à l'intérieur du périmètre de ladite zone optiquement active de la cornée, et à coordonner le fonctionnement dudit moyen (14) en vue de la déflexion de balayage dudit faisceau laser suivant un programme commandé de limitation d'un balayage de zone à l'intérieur d'une limite de périmètre, avant de répéter une telle coordination à l'intérieur de la limite de périmètre immédiatement consécutive, ce par quoi la pénétration d'ablation à la profondeur désirée est le résultat cumulé de plusieurs balayages de zone de chacune d'une succession de zones différentes mais se chevauchant.

11. Appareil selon la revendication 10, dans lequel ledit moyen (14) de déflexion de balayage dudit faisceau laser agit radialement par rapport à l'axe dudit faisceau au point d'incidence sur la cornée, et comprend un moyen pour faire pivoter la direction dans laquelle la déflexion radiale est active, ce par quoi, au cours d'une opération de balayage donnée, chaque balayage de zone est le résultat d'une course, développée en spirale, de déflexion du faisceau.

12. Appareil selon la revendication 10 ou 11, dans lequel lesdites limites de périmètre sont des limites extérieures circulaires de différentes zones circulaires balayées disposées de façon concentrique, et diminuant successivement, ce qui fait que le résultat cumulé du balayage en zones successives de la cornée est correcteur de myopie.

13. Appareil selon la revendication 10 ou 11, dans lequel lesdites limites de périmètre sont des limites circulaires de différentes zones annulaires balayées disposées de façon concentrique et de diamètre extérieur constant et avec des diamètres intérieurs augmentant successivement, ce qui fait que le résultat cumulé du balayage en zones successives de la cornée est correcteur d'hypermétropie.

14. Appareil selon l'une quelconque des revendications 1 à 9, dans lequel la limite du périmètre de balayage en zones successives est un cercle de rayon constant, ce qui fait que le résultat cumulé du balayage en zones successives de la cornée est de former une cavité cornéenne circulaire de profondeur constante en vue de la réception d'une greffe de cornée.

15. Appareil selon l'une quelconque des revendications 1 à 13, dans lequel ledit moyen de commande de laser (16) est conçu de telle sorte que des balayages en zones successives créent un développement étagé du type de Fresnel de la courbure finale désirée.

16. Appareil selon la revendication 15, dans lequel ledit moyen de commande de laser (16) est conçu pour commander le fonctionnement dudit moyen (14) de déflexion de balayage du faisceau laser dans chacune d'une pluralité d'aires de zones annulaires adjacentes, disposées de façon concentrique, et ledit moyen de commande de laser (16) est encore conçu pour assurer un balayage en zones successives de chaque aire de zone annulaire avec une variation du rayon de périmètre extérieur, à rayon de périmètre intérieur constant, l'appareil étant ainsi conçu pour produire un profil de surface cornéenne antérieure correcteur de myopie à caractéristique de Fresnel.

17. Appareil selon la revendication 15, dans lequel ledit moyen de commande de laser (16) est conçu pour commander le fonctionnement dudit moyen (14) de déflexion de balayage du faisceau laser dans chacune d'une pluralité d'aires de zones annulaires adjacentes, disposées de façon concentrique, et ledit moyen de commande de laser (16) est encore conçu en vue d'un balayage en zones successives de chaque aire de zone annulaire avec une variation du rayon de périmètre intérieur, à rayon de périmètre extérieur constant, l'appareil étant ainsi conçu pour produire un profil de surface cornéenne antérieure correcteur d'hypermétropie à caractéristique de Fresnel.

18. Appareil selon l'une quelconque des revendications 1 à 9, dans lequel ledit moyen (14) de déflexion de balayage du faisceau laser est adapté pour produire deux composantes orthogonales de déflexion contrôlée du faisceau transversalement à la direction de propagation du faisceau, et ledit moyen de commande de laser (16) est adapté pour coordonner le fonctionnement dudit moyen (14) de déflexion de balayage du faisceau laser suivant un programme contrôlé de couverture de zone pour établir la plus grande exposition au faisceau cumulée le long du prolongement de l'axe central de symétrie d'un état astigmate déterminé, avec une exposition au faisceau cumulée diminuant en fonction d'un décalage latéral croissant des deux côtés dudit axe central de symétrie, l'appareil étant ainsi adapté pour la correction d'un état astigmate.

19. Appareil selon la revendication 18, dans lequel l'exposition au faisceau cumulée est modifiée par variation de la vitesse de balayage dudit moyen de balayage du faisceau de sortie du laser.

# FIG. 1.

# FIG. 2.

# FIG. 3.

# FIG. 4.

# FIG. 5.

# FIG. 6.

FIG. 7.

FIG. 8.

FIG. 9.

FIG. 10.

FIG. 11.

POSTERIOR
CORNEAL
SURFACE·AXIS

mm RADIUS